# EUROPÄISCHE PATENTSCHRIFT

(11) **EP 2 383 012 B1**
(45) Veröffentlichungstag und Bekanntmachung des Hinweises auf die Patenterteilung: **05.06.2013**
(21) Anmeldenummer: 11161562.1
(22) Anmeldetag: 07.04.2011
(51) Int. Cl.: A61M 25/10

(54) **Kombinierter Rollmembran-Ballon-Katheter**
Combined rolling membrane-balloon catheter
Cathéter à ballonnet et à membrane enroulable combiné

(30) Priorität: 28.04.2010 US 328650 P
(43) Veröffentlichungstag der Anmeldung: 02.11.2011
(73) Patentinhaber: Biotronik AG, 8180 Bülach (CH)
(72) Erfinder: Wesselmann, Matthias, 8912, Glattfelden (CH); Lang, Hans, 8107, Buchs (CH); Pfenninger-Ganz, Susanne, 8607, Aathal (CH)
(74) Vertreter: Lindner-Vogt, Karin L.

(56) Entgegenhaltungen:
- CH-A5- 671 883
- US-A- 5 364 345
- US-A1- 2009 254 063

## Beschreibung

Die Erfindung betrifft einen kombinierten Rollmembran-Ballon-Katheter insbesondere zur Aufweitung von Stenosen in Körpergefäßen mit einem Außenschaft und einem axial darin verschiebbaren Innenschaft.

Aus der US 5,662,703 A ist ein kombinierter Rollmembran-Ballon-Katheter bekannt, der zum Ausbringen eines selbst expandierenden Stents im Bereich einer Stenose in einem Körpergefäß ausgelegt ist. Dieser Katheter weist einen Innen- und Außenschaft auf, zwischen deren distalen Enden eine auf sich selbst gestülpte Rollmembran angesetzt ist, die das distale Ende des Katheters bildet. Diese Rollmembran bildet einen mit dem Katheter koaxial verlaufenden Raum vor dem distalen Ende des Innenschaftes, in dem der selbstexpandierende Stent in seinem kontrahierten Zustand untergebracht ist.

Proximal vor dem distalen Ende des Innenschaftes ist ein durch Druckbeaufschlagung expandierbarer Ballon angeordnet, der zur Unterstützung der Expansionsbewegung des Stents dient.

Zu dessen Freisetzung wird der Außenschaft in proximaler Richtung relativ zum Innenschaft bewegt, wodurch sich die auf sich selbst gestülpte Rollmembran in einer schälenden Bewegung nach hinten vom Stent abzieht und diesen allmählich freisetzt. Dieser weitet sich durch seine Selbstexpansionswirkung allmählich auf, bis er durch komplettes Abschälen der Rollmembran nach hinten frei wird. Gleichzeitig wird die Rollmembran dabei in proximaler Richtung über den Ballon gezogen, der nun unmittelbar vor dem distalen Ende des Katheters liegt. Dieser kann in den expandierten Stent in axialer Richtung eingeschoben und der Ballon anschließend durch Druckbeaufschlagung expandiert werden. Dadurch wird der Stent optimal aufgeweitet und im Körpergefäß vorhandene Stenosen zusätzlich mechanisch aufgeweitet. Da der Ballon dabei innerhalb der Rollmembran liegt, ist die gesamte Anordnung besonders druckdicht. Bei einem Bersten des Ballons verhindert die um ihn herum liegende Rollmembran ein Austreten des unter hohem Druck stehenden Druckfluids in das Körpergefäß. Der Druck wird vielmehr über den Innenraum des Außenkatheters entlastet.

Siehe ebenso US 5 364 345

Aus der US 5,593,418 A ist eine Vorrichtung zur Entnahme von Venenstücken bekannt, bei der eine durch ein Druckfluid über die zu entnehmende Vene ausstülpbare Rollmembran vorgesehen ist. Ferner weist diese Vorrichtung einen Verankerungsballon auf, der vor dem Ausstülpen der Rollmembran innerhalb der Vene aufgeblasen wird, um diese gegenüber der Rollmembran zu fixieren.

Zum Hintergrund der Erfindung ist ferner darauf hinzuweisen, dass zur Behandlung von Stenosen in Körpergefäßen an die dabei eingesetzten Katheter grundsätzliche Forderungen zu stellen sind. So soll ein möglichst reibungsfreies Eindringen auch in enge, stark gewundene Läsionen möglich sein, dennoch sollen zur Erzielung hoher Aufweitungskräfte die dabei verwendeten Ballone mit hohem Druck beaufschlagbar sein. Sie müssen entsprechend hoch druckfest sein, womit sie in der Regel von ihrer Materialbeschaffenheit her biegesteifer werden. Dies bedingt wiederum Probleme beim Eindringen in enge, gewundene Körpergefäße.

In dem vorstehenden Spannungsfeld verschiedener Probleme sind einerseits Rollmembrankatheter grundsätzlich bekannt, die zwar sehr reibungsfrei in eine Stenose oder stark gewundene Gefäßbereiche eindringen können, aufgrund ihrer notwendigen Überstülpung sind solche Rollmembranen jedoch nur schwierig mit einem Ballonlumen druckfest zu verbinden.

Ferner sind hydrophile Beschichtungen von konventionellen Ballonkathetern bekannt, die zwar deren Reibwert beträchtlich herabsetzen, bei stark gewundenen, langen Läsionen kann ein solcher Katheter dennoch steckenbleiben. Es muss also in der Regel ein Kompromiss aus Flexibilität und Schiebbarkeit des Katheters gefunden werden, der nicht für alle klinischen Fälle optimal sein kann.

Schließlich ist ein weiteres grundsätzliches Problem bei solchen Ballonkathetern, dass diese an ihrem Therapieort, also im Bereich etwa einer Stenose, in der Regel nicht verankert sind.

Ausgehend von den geschilderten Problemen des Standes der Technik liegt der Erfindung die Aufgabe zugrunde, einen kombinierten Rollmembran-Ballon-Katheter so weiterzubilden, dass er unter möglichst weitgehender Ausschaltung von Reibung und mit geringer Biegesteifigkeit einfacher in problematische Körpergefäßzonen einführbar ist, dort jedoch unter Verankerung vor einer Stenose eine hohe Dilatierkraft auf sichere Weise erzeugt.

Diese Aufgabe wird durch die im Kennzeichnungsteil des Anspruchs 1 angegebenen Merkmale gelöst, die umfassen
- einen zwischen Innen- und Außenschaft angeordneten, ebenfalls axial verschiebbaren Zwischenschaft,
- eine zwischen distalem Ende des Außenschaftes und distalem Ende des Innenschaftes druckdicht befestigte Rollmembran, die zwischen einer Passivstellung innerhalb des Außenschaftes und einer distal aus dem Außenschaft durch Druckbeaufschlagung expandierten Aktivstellung verlagerbar ist, sowie
- einen zwischen distalem Ende des Innenschaftes und distalem Ende des Zwischenschaftes druckdicht befestigten, dilatierbaren Ballon, der zwischen einer Passivstellung innerhalb des Außenschaftes und proximal vor der Rollmembran einerseits und einer distal aus dem Außenschaft, innerhalb der Rollmembran durch Druckbeaufschlagung expandierten Aktivstellung andrerseits verlagerbar ist.

Der erfindungsgemäße Katheter stellt also eine Synthese aus einem Rollmembran- und einem konventionellen Ballonkatheter dar, der folgende Vorteile aufweist:
- Die Reibung wird durch das Abrollen der außen liegenden Rollmembran und die Flüssigkeitsreibung zwischen der Membran und dem Ballon aufgrund des verwendeten Druckfluids für die Druckbeaufschlagung minimiert.
- Der Katheter wird durch das Inflatieren der sich in die Aktivstellung expandierenden Rollmembran vor der Stenose verankert.
- Die beim Übergang zwischen der Passiv- und Aktivstellung der Rollmembran in distaler Richtung austretende Wand erzeugt eine Vorschubkraft, die proportional zum Querschnitt dieser distalen Wand und dem hydraulischen Druck ist. Die Druckkraft wird damit verlustfrei zum Applikationsort im Bereich der Stenose verbracht.
- Die Biegesteifigkeit des kombinierten Rollmembran-Ballon-Katheters wird dadurch gesenkt, dass beim Einführen des Katheters zum Bereich einer Stenose die Rollmembran und der Ballon hintereinander innerhalb des Katheter-Außenschaftes liegen, in radialer Richtung also nur minimal auftragen. Zum Passieren der Stenose muss sich nur noch der Ballon verbiegen.
- Da die Rollmembran und der in Aktivstellung sich darin befindliche Ballon zusammen einen sogenannten Doppelmembranballon bilden, kann diese Formation deutlich höhere Druckwerte aushalten. Insgesamt kann die Ballonwand also dünner ausgeformt werden, was die Biegesteifigkeit der Anordnung wiederum herabsetzt. Außerdem ist der Konus des Ballons wegen der reduzierten Wandstärke weicher.
- Das beschriebene Verfahren zur Herstellung eines Doppelballonkatheters ist besonders vorteilhaft in der Fertigung im Vergleich zu bekannten Doppelballonkatheteraufbauten. Dort werden zwei Ballone nacheinander geformt und dann der innere Ballon in den engen Hals des äußeren Ballons montiert.
- Der Ballon selbst entspricht in seiner Anordnung bekannten Konstruktionen, die bewährt und deren Druckfestigkeit damit sichergestellt ist. Die außenseitige Rollmembran stützt dabei zusätzlich den innenliegenden Ballon.

Die Erfindung betrifft ferner ein Verfahren zur Herstellung eines solchem kombinierten Rollmembran-Ballon-Katheters gemäß Anspruch 10, das durch folgende Verfahrensschritte charakterisiert ist:
- Herstellen zweier axial hintereinander liegender Ballonrohlinge aus einem Schlauchstück,
- Befestigen des proximalen Endes des proximalen Ballonrohlings am distalen Ende des Katheter-Zwischenschaftes,
- Befestigen des distalen Endes des proximalen Ballonrohlings am distalen Ende des Innenschaftes,
- Einführen der Baugruppe aus den Ballonrohlingen sowie dem Innen- und Zwischenschaft in den Außenschaft, und
- Befestigen des distalen Endes -des als Rollmembran fungierenden distalen Ballonrohlings am distalen Ende des Außenschaftes.

Alternativ können die zwei Ballonrohlinge durch Thermoformung aus einem einzigen Schlauchstück oder auch getrennt ausgeformt werden. Die beiden Ballonrohlinge können dann an Innen-, Zwischen- und/oder Außenschaft des Katheters durch Schweißen oder Kleben befestigt werden.

Bevorzugte Ausführungsformen der Erfindung sind in den abhängigen Ansprüchen angegeben. Merkmale, Einzelheiten und Vorteile hierzu ergeben sich aus der nachfolgenden Beschreibung eines Ausführungsbeispiels anhand der beigefügten Zeichnungen. Es zeigen:
- Fig. 1: einen schematischen Axialschnitt durch einen Rollmembran-Ballon-Katheter,
- Fig. 2A bis 2E: schematische Darstellungen der Herstellung des Katheters nach Fig. 1 in aufeinander folgenden Fertigungsschritten, und
- Fig. 3 bis 13: schematische Längsschnitte durch ein Köpergefäß im Bereich einer Stenose mit einem Rollmembran-Ballon-Katheter in aufeinander folgenden Therapieschritten.

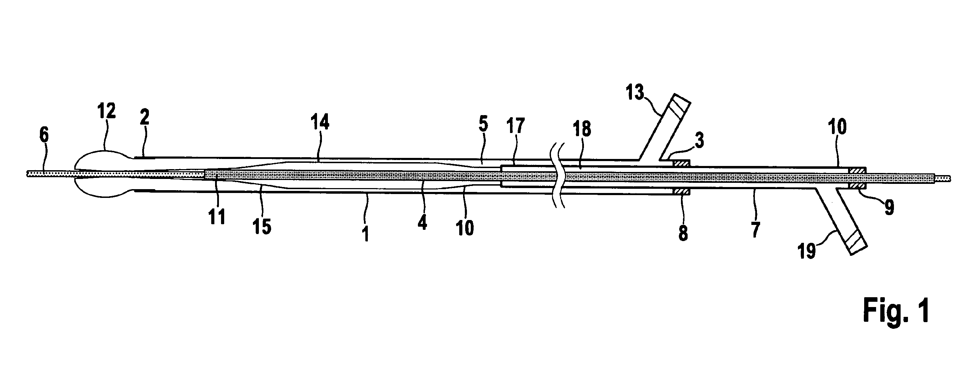

Wie aus Fig. 1 hervorgeht, weist der dargestellte Rollmembran-Ballon-Katheter einen lang gestreckten Außenschaft 1 mit einem distalen Ende 2, mit dem er in ein Körpergefäß einführbar ist, und einem proximalen Ende 3 auf. In dem Außenschaft 1 ist ein Innenschaft 4 angeordnet, der demgegenüber einen deutlich geringeren Durchmesser aufweist und dementsprechend zusammen mit dem Außenschaft 1 einen Ringraum 5 bildet. Im Innenschaft 4 ist ein nicht näher dargestelltes Lumen angeordnet, mit dem der Katheter über einen Führungsdraht 6 - einem sogenannten "Guide Wire" - zum Therapieort in einem Körpergefäß geschoben werden kann.

Zwischen Außenschaft 1 und Innenschaft 4 ist ferner ein Zwischenschaft 7 koaxial zu den beiden vorgenannten Schäften 1, 4 angeordnet, der mit dem Innenschaft 4 axial gegenüber dem Außenschaft 1 verschiebbar ist. Dazu ist der Zwischenschaft 7 in einer den Ringraum 5 proximal druckdicht verschließenden Ringdichtung 8 in Form beispielsweise einer sogenannten "Tuhoy Borst"-Dichtung verschiebbar. Der Innenschaft 4 wiederum ist druckdicht durch einen Abschlussstopfen 9 am proximalen Ende 10 geführt.

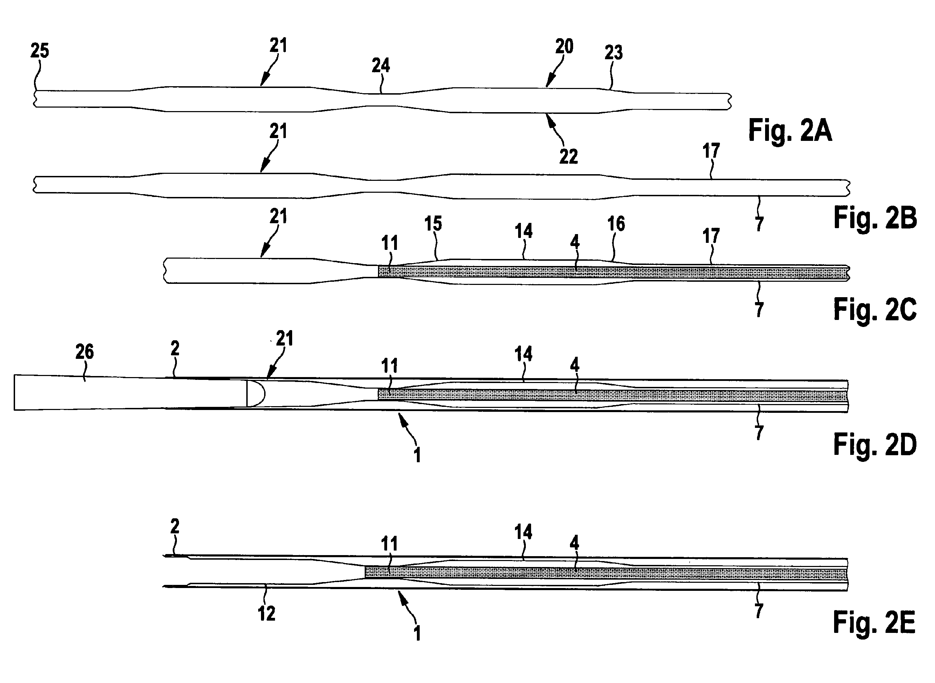

Zwischen dem distalen Ende 2 des Außenschafts 1 und dem distalen Ende 11 des Innenschafts 4 ist eine sogenannte Rollmembran 12 angeordnet, die in Fig. 1 in einer bereits leicht aus dem Außenschaft 1 ausgeschobenen Position dargestellt ist. Die eigentliche Passivstellung der Rollmembran 12 innerhalb des Außenschafts 1 ist in Fig. 2E erkennbar. Die Rollmembran 12 ist druckdicht mit dem Außenschaft 1 und Innenschaft 2 über entsprechende Verschweißungen verbunden und kann über den Ringraum 5 mit einem Druckfluid beaufschlagt werden, das durch den Membran-Druckstutzen 13 am proximalen Ende 3 des Außenschafts 1 unter Druck eingeführt werden kann.

Proximal vor der Rollmembran 12 ist innerhalb des Außenschafts 1 ferner ein Ballon 14 angeordnet, dessen distaler Konus 15 am distalen Ende 11 des Innenschafts 4 druckdicht befestigt ist. Der proximale Konus des Ballons 14 wiederum ist druckdicht am distalen Ende 17 des Zwischenschafts 7 angebracht. Der Ballon 14 ist über den zwischen Innenschaft 4 und Zwischenschaft 7 gebildeten Ringraum 18 mit einem Druckfluid beaufschlagbar, das über einen Ballon-Druckstutzen 19 am proximalen Ende 10 des Zwischenschafts 7 einführbar ist.

Anhand von Fig. 2A bis 2E kann die Herstellung des kombinierten Rollmembran-Ballon-Katheters gemäß Fig. 1 näher erläutert werden. So werden ausgehend von einem einzigen durchgehenden Schlauchstück 20 durch Thermoformen axial hintereinander liegend zwei Ballonrohlinge 21, 22 durch Thermoformen gebildet, wobei der erste Ballonrohling 21 später die Rollmembran 12 und der zweite Ballonrohling 22 später den eigentlichen Ballon 14 bilden (Fig. 2A). Das proximale Ende 23 des proximalen Ballonrohlings 22 wird an das distale Ende 17 des Zwischenschafts 7 beispielsweise durch Verschweißen befestigt (Fig. 2B).

Anschließend wird das Schlauchstück 20 im Übergangsbereich zwischen den beiden Ballonrohlingen 21, 22 am distalen Ende 11 des Innenschafts 4 beispielsweise wiederum durch Schweißen befestigt, sodass das distale Ende 24 des proximalen Ballonrohlings 22 dort fest gelegt ist. Damit ist aus dem Ballonrohling 22 der Ballon 14 des Katheters definiert (siehe Fig. 2C).

Im nächsten Fertigungsschrift wird die gesamte Baugruppe aus den Ballonrohlingen 21, 22, dem Innenschaft 4 und dem Zwischenschaft 7 in den Außenschaft 1 eingeführt und das distale Ende 25 des distalen Ballonrohlings 21 am distalen Ende 2 des Außenschafts 7 durch Schweißen oder Kleben mithilfe eines in den Außenschaft 1 eingeführten Werkzeugs 26 befestigt (Fig. 2D).

Es ergibt sich damit der fertige Rollmembran-Ballon-Katheter, wie er in der Passivstellung des Ballons 14 und der Rollmembran 12 innerhalb des Außenschafts 1 in Fig. 2E dargestellt ist.

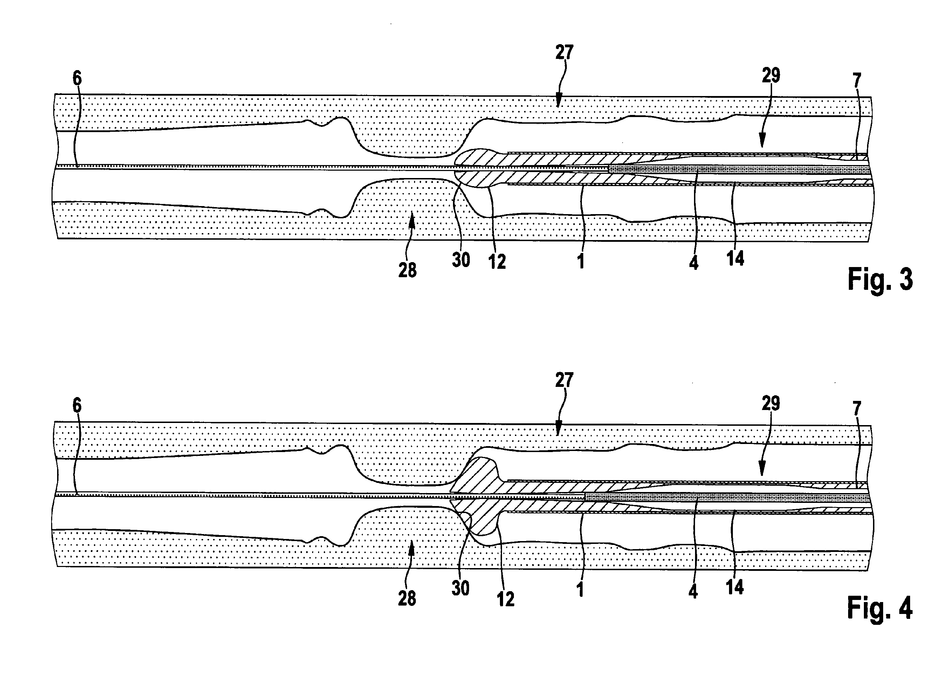

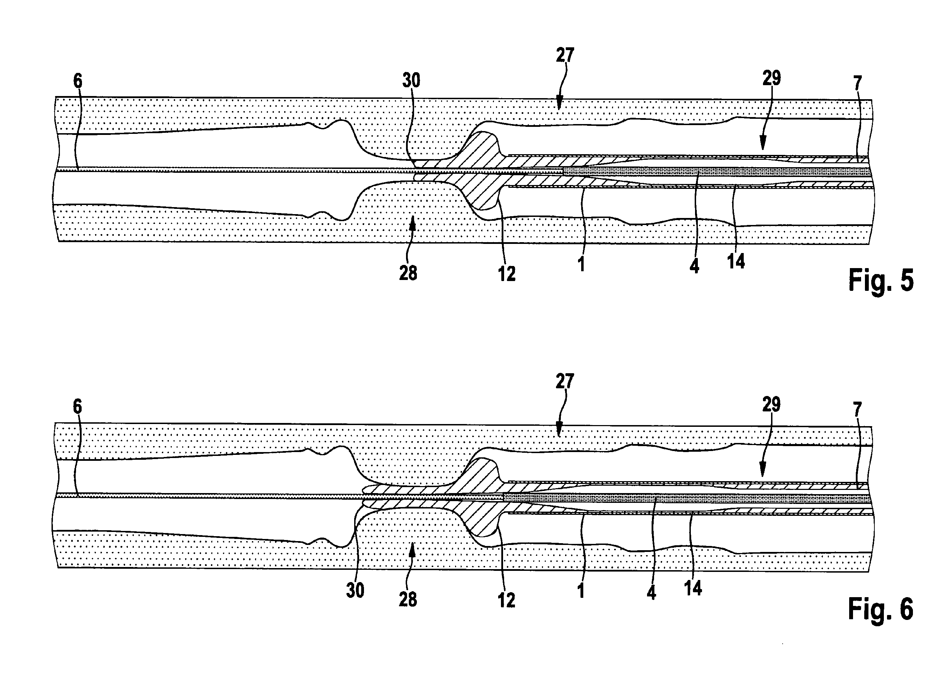

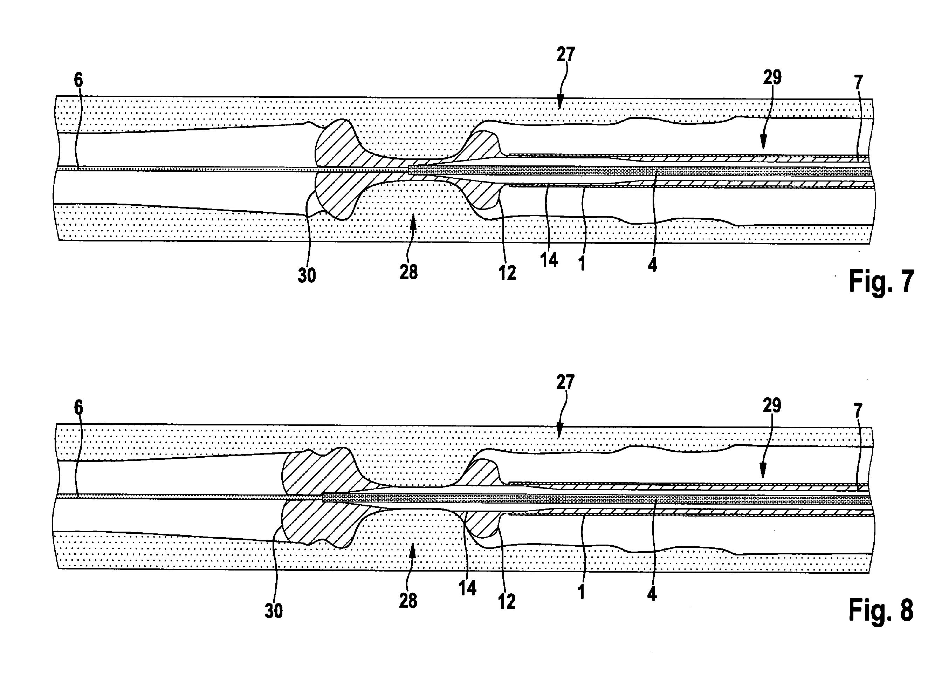

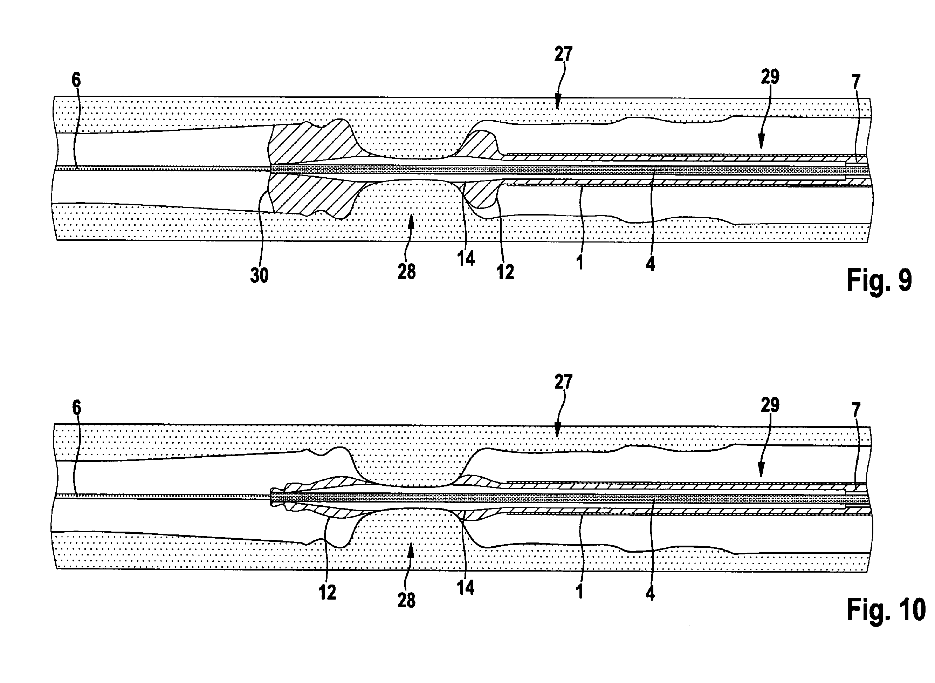

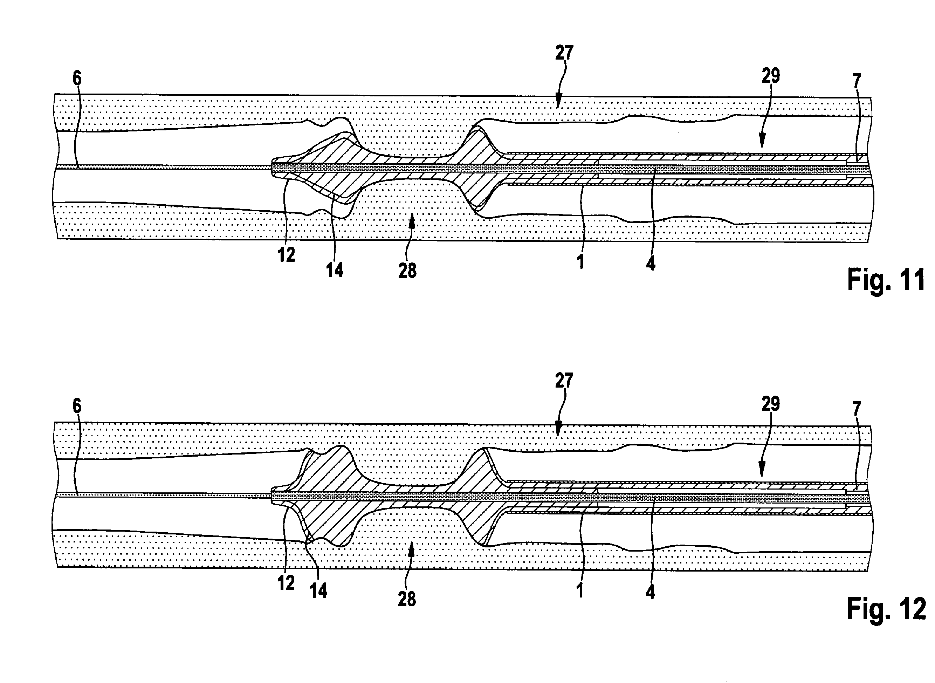

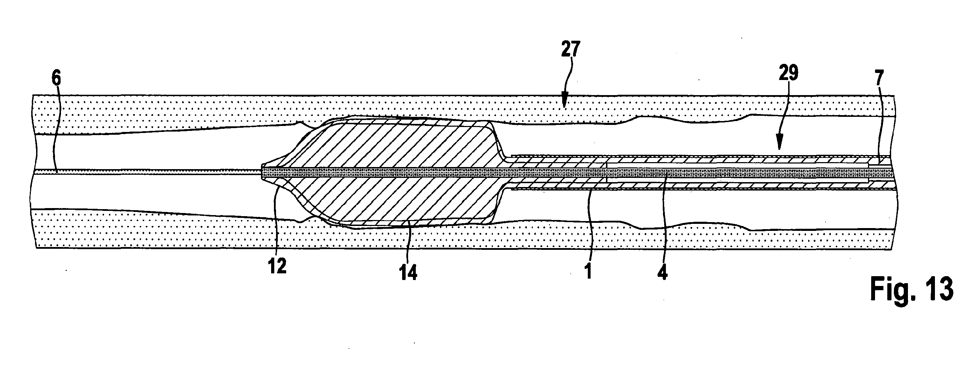

Anhand der Fig. 3 bis 13 ist die klinische Anwendung des kombinierten Rollmembran-Ballon-Katheters 29 zu erläutern. So zeigen diese Figuren ein Körpergefäß 27 mit einer krankhaften Verengung in Form einer Stenose 28. Der Katheter 29 wird auf dem vorher durch die Stenose 28 geführten Führungsdraht 6 bis an die Stenose 28 herangeschoben (Fig. 3). In dieser Passivstellung der Rollmembran 12 wird über den Membran-Druckstutzen 13 ein Druckfluid in den Ringraum 5 zwischen Innen- und Außenschaft 4, 1 eingeführt, das am zusammengefalteten Ballon 14 vorbei für ein Inflatieren der Rollmembran 12 sorgt. Indem Innenschaft 4 und Zwischenschaft 7 gemeinsam in distaler Richtung bewegt werden, kann bei diesem Inflatieren die Rollmembran 12 aus dem Außenschaft 1 distal nach vorne auf dem Führungsdraht 6 in ihre Aktivstellung austreten und in radialer Richtung expandieren (Fig. 4). Dadurch erfolgt ein Verankern des Katheters 29 vor der Stenose 28. Bei der weiteren Druckbeaufschlagung dringt die voreilende Wand der Rollmembran 12 in den Bereich zwischen Führungsdraht 6 und Stenose 28 ein, da der hydraulische Druck auf diese distale Wand 30 für eine Vorschubkraft vor Ort sorgt. Diese zieht den Innenschaft 4 weiter mit (Fig. 5), bis die Rollmembran 12 die Stenose 28 passiert (Fig. 6) und sich dahinter ebenfalls in radialer Richtung aufweitet (Fig. 7). Dabei wird der Innenschaft 4 zusammen mit dem Zwischenschaft 7 und dem Ballon 14 immer weiter aus dem Außenschaft 1 heraus und allmählich ebenfalls in den Bereich der Stenose 28 gezogen. Wie aus den Fig. 8 und 9 deutlich wird, setzt sich diese Bewegung fort, bis auch der Ballon 14 aus seiner Passivstellung innerhalb des Außenschafts 1 komplett in die in Fig. 9 gezeigte Aktivstellung außerhalb des Außenschafts 1 zentral in der Stenose 28 platziert ist. In dieser Position wird die Druckbeaufschlagung der Rollmembran 12 entlastet, sodass auch auf den Ballon 14 kein Druck mehr von radial außen wirkt (Fig. 10). Damit ist die Voraussetzung dafür geschaffen, dass durch Einführen eines Druckfluids über den Ballon-Druckstutzen 19 der Ballon 14 mit Druck beaufschlagt werden kann. Dieser dilatiert über seine gesamte Länge und weitet so die Stenose 28 in radialer Richtung komplett auf, wie dies aus einer Zusammenschau der Fig. 11 bis 13 deutlich wird.

Nach einem Entlasten des Ballons 14 kann der gesamte Katheter 29 wieder aus dem Körpergefäß 27 herausgezogen werden.

Aus der vorstehenden Beschreibung der medizinischen Anwendung des Katheters werden verschiedene Vorteile nochmals deutlich:
- Die Haft- und Gleitreibung bei üblichen Rollmembran-Konstruktionen wird beim erfindungsgemäßen Katheter durch eine Rollreibung innerhalb der Rollmembran 12 ersetzt.
- Wie aus den Fig. 5 bis 8 deutlich wird, kann sich der Katheter mit seiner Rollmembran 12 auch in enge Spalte ohne nennenswerte Reibung hineinzwängen.
- Am Therapieort ist eine bessere Vorschiebbarkeit, also eine hohe sogenannte "Pushability", aufgrund des hydraulischen Vortriebs der Rollmembran zu erzielen. Das System erzeugt seine Vorschubkraft an der Stelle, wo sie benötigt wird; es gibt keine Reibungsverluste entlang des Schafts selbst.
- Die Rollmembran kann sich vor der Stenose verankern, wodurch die Eindringfähigkeit des Therapiegeräts in die Stenose weiter verbessert wird.
- Der Katheter ist durch die Hintereinanderanordnung von Rollmembran 12 und Ballon 14 während des Einführens besser entlang des Führungsdrahts schiebbar.
- Der Katheter weist eine geringere Empfindlichkeit gegen Leckeffekte, wie sogenannte "Pin Holes" auf.
- Die klinische Anwendung des Geräts ist vergleichsweise einfach und logisch aufgeschlüsselt, was den Ablauf der einzelnen Schritte anbetrifft.

## Patentansprüche

1. Kombinierter Rollmembran-Ballon-Katheter, insbesondere zur Aufweitung von Stenosen in Körpergefäßen, umfassend
- einen Außenschaft (1), und
- einen darin axial verschiebbaren Innenschaft (4),
- einen zwischen Innen- und Außenschaft (4; 1) angeordneten, ebenfalls axial verschiebbaren Zwischenschaft (7),
- eine zwischen distalem Ende (2) des Außenschaftes (1) und distalem Ende (11) des Innenschaftes (4) druckdicht befestigte Rollmembran (12), die zwischen einer Passivstellung innerhalb des Außenschaftes (1) und einer distal aus dem Außenschaft (1) durch Druckbeaufschlagung expandierten Aktivstellung verlagerbar ist, **gekennzeichnet durch**
- einen zwischen distalem Ende (11) des Innenschaftes (4) und distalem Ende des Zwischenschaftes (7) druckdicht befestigten, dilatierbaren Ballon (14), der zwischen einer Passivstellung innerhalb des Außenschaftes (1) und proximal vor der Rollmembran (12) einerseits und einer distal aus dem Außenschaft (1), innerhalb der Rollmembran (12) **durch** Druckbeaufschlagung expandierten Aktivstellung andrerseits verlagerbar ist.

2. Rollmembran-Ballon-Katheter nach Anspruch 1, **dadurch gekennzeichnet, dass** der Innenschaft (4) ein Lumen für einen Führungsdraht (6) zur Positionierung des Katheters aufweist.

3. Rollmembran-Ballon-Katheter nach Anspruch 1 oder 2, **dadurch gekennzeichnet, dass** der Innenschaft (4) während der Expansion der Rollmembran (12) aus dem Außenschaft (1) fortschreitend unter Mitnahme des Ballons (14) mit dem Zwischenschaft (7) in die sich bei der Expansion ausstülpende Rollmembran (12) einschiebbar ist.

4. Rollmembran-Ballon-Katheter nach einem der vorgenannten Ansprüche, **dadurch gekennzeichnet, dass** zur Druckbeaufschlagung der Rollmembran (12) der Ringraum (5) zwischen dem Zwischen- und Außenschaft (7; 1) als Druckfluidleitung vorgesehen ist, der proximal an einen Membran-Druckstutzen (13) am Außenschaft (1) angeschlossen ist.

5. Rollmembran-Ballon-Katheter nach einem der vorgenannten Ansprüche, **dadurch gekennzeichnet, dass** zur Druckbeaufschlagung des Ballons (14) der Ringraum (5) zwischen dem Zwischen- und Innenschaft (7; 4) als Druckfluidleitung vorgesehen ist, der proximal an einen Ballon-Druckstutzen (19) am Zwischenschaft (7) angeschlossen ist.

6. Rollmembran-Ballon-Katheter nach einem der vorgenannten Ansprüche, **dadurch gekennzeichnet, dass** der Zwischenschaft (7) am proximalen Ende (3) des Außenschafts (1) in einer druckdichten Ringdichtung (8) axial verschiebbar geführt ist.

7. Rollmembran-Ballon-Katheter nach einem der vorgenannten Ansprüche, **dadurch gekennzeichnet, dass** die Rollmembran (12) und der Ballon (14) gemeinsam aus einem einzigen schlauchförmigen Stück bestehen.

8. Rollmembran-Ballon-Katheter nach einem der vorgenannten Ansprüche, **dadurch gekennzeichnet, dass** die Rollmembran (12) beim Übergang von ihrer Passiv- in die Aktivstellung in einer Ausstülpbewegung nach distal überlagert mit einer radialen Ausdehnbewegung expandierbar ist.

9. Rollmembran-Ballon-Katheter nach einem der vorgenannten Ansprüche, **dadurch gekennzeichnet, dass** die Rollmembran (12) vor der Expansion des Ballons (14) deflatierbar ist.

10. Verfahren zur Herstellung eines kombinierten Rollmembran-Ballon-Katheters nach einem der vorgenannten Ansprüche, **gekennzeichnet durch** folgende Verfahrensschritte:
- Herstellen zweier axial hintereinander liegender Ballonrohlinge (21, 22) aus einem Schlauchstück (20),
- Befestigen des proximalen Endes (23) des proximalen Ballonrohlings (22) am distalen Ende des Katheter-Zwischenschaftes (7),
- Befestigen des distalen Endes (17) des proximalen Ballonrohlings am distalen Ende des Innenschaftes (4),
- Einführen der Baugruppe aus den Ballonrohlingen (21, 22) sowie dem Innen- und Zwischenschaft (4; 7) in den Außenschaft (1), und
- Befestigen des distalen Endes (25) des als Rollmembran (12) fungierenden distalen Ballonrohlings (21) am distalen Ende (2) des Außenschaftes (1).

11. Verfahren nach Anspruch 10, **dadurch gekennzeichnet, dass** die zwei Ballonrohlinge (21, 22) durch Thermoformung aus einem einzigen Schlauchstück (20) hergestellt werden.

12. Verfahren nach Anspruch 10, **dadurch gekennzeichnet, dass** die zwei Ballonrohlinge getrennt ausgeformt werden.

13. Verfahren nach einem der Ansprüche 10 bis 12, **dadurch gekennzeichnet, dass** die beiden Ballonrohlinge (21, 22) an Innen-, Zwischen- und/oder Außenschaft (4; 7; 1) durch Schweißen oder Kleben befestigt werden.

## Claims

1. A combined rolling membrane/balloon catheter, in particular for expanding stenoses in bodily vessels, comprising:
- an outer shaft (1), and
- an inner shaft (4) axially displaceable therein,
- an intermediate shaft (7) situated between the inner and outer shaft (4; 1) and likewise axially displaceable,
- a rolling membrane (12), which is fastened in a pressure-tight manner between the distal end (2) of the outer shaft (1) and the distal end (11) of the inner shaft (4), and which may be displaced, by the action of pressure, between a passive position within the outer shaft (1) and an active position distally expanded from the outer shaft (1),
**characterised by**
- a dilatable balloon (14), which is fastened in a pressure-tight manner between the distal end (11) of the inner shaft (4) and the distal end of the intermediate shaft (7), and which may be displaced, by the action of pressure, between a passive position within the outer shaft (1) and proximally in front of the rolling membrane (12), and an active position distally expanded from the outer shaft (1), within the rolling membrane (12).

2. The rolling membrane/balloon catheter according to Claim 1, **characterised in that** the inner shaft (4) comprises a lumen for a guide wire (6) for positioning the catheter.

3. The rolling membrane/balloon catheter according to Claim 1 or 2, **characterised in that**, during expansion of the rolling membrane (12) from the outer shaft (1), the inner shaft (4), while carrying along the balloon (14) together with the intermediate shaft (7), can be progressively inserted into the rolling membrane (12), which is unrolling during the expansion.

4. The rolling membrane/balloon catheter according to one of the preceding claims, **characterised in that**, for pressure impingement on the rolling membrane (12), the annular space (5) between the intermediate shaft and the outer shaft (7; 1) is provided as a pressure fluid line, which is proximally connected to a membrane pressure joint (13) on the outer shaft (1).

5. The rolling membrane/balloon catheter according to one of the preceding claims, **characterised in that**, for the pressure impingement of the balloon (14), the annular space (5) between the intermediate shaft and the inner shaft (7; 4) is provided as a pressure fluid line, which is proximally connected to a balloon pressure joint (19) on the intermediate shaft (7).

6. The rolling membrane/balloon catheter according to one of the preceding claims, **characterised in that** the intermediate shaft (7) is guided in an axially displaceable manner in a pressure-tight ring seal (8) at the proximal end (3) of the outer shaft (1).

7. The rolling membrane/balloon catheter according to one of the preceding claims, **characterised in that** the rolling membrane (12) and the balloon (14) both consist of a single tubular portion.

8. The rolling membrane/balloon catheter according to one of the preceding claims, **characterised in that**, during transition from its passive to its active position, the rolling membrane (12) may be expanded in an unrolling motion, in distal direction superimposed with a radial expansion motion.

9. The rolling membrane/balloon catheter according to one of the preceding claims, **characterised in that** the rolling membrane (12) may be deflated before the balloon (14) is expanded.

10. A method for producing a combined rolling membrane/balloon catheter according to one of the preceding claims, **characterised by** the following method steps:
- producing two balloon blanks (21, 22), which are axially positioned one behind the other, from a portion of tubing (20),
- fastening the proximal end (23) of the proximal balloon blank (22) to the distal end of the catheter intermediate shaft (7),
- fastening the distal end (17) of the proximal balloon blank to the distal end of the inner shaft (4),
- inserting the assembly composed of the balloon blanks (21, 22) and the inner shaft and intermediate shaft (4; 7) into the outer shaft (1), and
- fastening the distal end (25) of the distal balloon blank (21), which functions as a rolling membrane (12), to the distal end (2) of the outer shaft (1).

11. The method according to Claim 10, **characterised in that** the two balloon blanks (21, 22) are produced by thermoforming from a single portion of tubing (20).

12. The method according to Claim 10, **characterised in that** the two balloon blanks are formed separately.

13. The method according to one of Claims 10 to 12, **characterised in that** the two balloon blanks (21, 22) are fastened to the inner, intermediate, and/or outer shaft (4; 7; 1) by welding or gluing.

## Revendications

1. Cathéter combiné à ballon et à membrane enroulée, en particulier pour l'élargissement de sténoses dans des vaisseaux corporels, comprenant
- une tige extérieure (1), et
- une tige intérieure (4) déplaçable axialement dans celle-ci,
- une tige intermédiaire (7) également déplaçable axialement, disposée entre la tige intérieure et la tige extérieure (4 ; 1)
- une membrane enroulée (12) fixée de manière étanche à la pression entre l'extrémité distale (2) de la tige extérieure (1) et l'extrémité distale (11) de la tige intérieure (4), tout en étant déplaçable entre une position passive à l'intérieur de la tige extérieure (1) et une position active expansée de façon distale hors de la tige extérieure (1) par application d'une pression,
**caractérisé par**
- un ballon dilatable (14) fixé de manière étanche à la pression entre l'extrémité distale (11) de la tige intérieure (4) et l'extrémité distale de la tige intermédiaire (7), tout en étant déplaçable entre une position passive à l'intérieur de la tige extérieure (1) et proximale devant la membrane enroulée (12) d'une part, et une position active expansée à l'intérieur de la membrane enroulée (12) de façon distale hors de la tige extérieure (1) par application d'une pression d'autre part.

2. Cathéter à ballon et à membrane enroulée selon la revendication 1, **caractérisé en ce que** la tige intérieure (4) présente un lumen pour un fil de fer de guidage (6), en vue du positionnement du cathéter.

3. Cathéter à ballon et à membrane enroulée selon la revendication 1 ou 2, **caractérisé en ce que** pendant l'expansion de la membrane enroulée (12), la tige intérieure (4) peut être insérée dans la membrane enroulée (12) faisant saillie lors de l'expansion, tout en sortant progressivement de la tige extérieure (1) et en entraînant le ballon (14) avec la tige intermédiaire (7).

4. Cathéter à ballon et à membrane enroulée selon l'une des revendications précédentes, **caractérisé en ce que** pour l'application d'une pression sur la membrane enroulée (12), l'espace annulaire entre la tige intermédiaire et la tige extérieure (7 ; 1) sert à guider le fluide sous pression, tout en étant raccordé de façon proximale à un tuyau de refoulement (13) appartenant à la membrane, sur la tige extérieure (1).

5. Cathéter à ballon et à membrane enroulée selon l'une des revendications précédentes, **caractérisé en ce que** pour l'application d'une pression sur le ballon (14), l'espace annulaire entre la tige intermédiaire et la tige intérieure (7 ; 4) sert à guider le fluide sous pression, tout en étant raccordé de façon proximale à un tuyau de refoulement (19) appartenant au ballon, sur la tige intermédiaire (7).

6. Cathéter à ballon et à membrane enroulée selon l'une des revendications précédentes, **caractérisé en ce que** la tige intermédiaire (7) est guidée dans un joint annulaire de façon à pouvoir être déplacée axialement, à l'extrémité proximale (3) de la tige extérieure (1).

7. Cathéter à ballon et à membrane enroulée selon l'une des revendications précédentes, **caractérisé en ce que** la membrane enroulée (12) et le ballon (14) sont constitués d'une seule pièce en forme de tube.

8. Cathéter à ballon et à membrane enroulée selon l'une des revendications précédentes, **caractérisé en ce que** lors de son passage de la position passive à la position active, la membrane enroulée (12) peut être expansée avec un mouvement de déploiement dans la direction distale superposé par un mouvement d'expansion radial.

9. Cathéter à ballon et à membrane enroulée selon l'une des revendications précédentes, **caractérisé en ce que** la membrane enroulée (12) peut être dégonflée avant l'expansion du ballon (14).

10. Procédé pour la fabrication d'un cathéter à ballon et à membrane enroulée selon l'une des revendications précédentes, **caractérisé par** les étapes de procédé suivantes :
- fabrication de deux pièces brutes de ballon (21, 22) disposées l'une à la suite de l'autre dans le sens axial, à partir d'un seul morceau de tuyau,
- fixation de l'extrémité proximale (23) de la pièce brute de ballon proximale (22) à l'extrémité distale de la tige intermédiaire (7) du cathéter,
- fixation de l'extrémité distale (17) de la pièce brute de ballon proximale à l'extrémité distale de la tige intérieure (4),
- insertion du bloc de construction constitué des pièces brutes de ballon (21, 22) et des tiges intérieure et intermédiaire (4 ; 7) dans la tige extérieure (1), et
- fixation de l'extrémité distale (25) de la pièce brute de ballon (21) servant de membrane enroulée (12) à l'extrémité distale (2) de la tige extérieure (1).

11. Procédé selon la revendication 10, **caractérisé en ce que** les deux pièces brutes de ballon (21, 22) sont réalisées par thermoformage à partir d'un seul morceau de tuyau (20).

12. Procédé selon la revendication 10, **caractérisé en ce que** les deux pièces brutes de ballon sont formées séparément.

13. Procédé selon l'une des revendications 10 à 12, **caractérisé en ce que** les deux pièces brutes de ballon (21, 22) sont fixées par soudage ou collage à la tige intérieure, à la tige intermédiaire et/ou à la tige extérieure (4 ; 7 ; 1).
